# EUROPEAN PATENT APPLICATION

(11) **EP 4 714 484 A1**
(43) Date of publication of application: **25.03.2026**
(21) Application number: 24839819.0
(22) Date of filing: 12.07.2024
(51) Int. Cl.: A61M 25/00

(54) **CATHETER, TREATMENT METHOD, GUIDE WIRE, AND CATHETER SYSTEM**

(30) Priority: 12.07.2023 JP 2023114344
(71) Applicant: Terumo Kabushiki Kaisha, Tokyo 151-0072 (JP)
(72) Inventor: ITO Takashi, Fujinomiya-shi, Shizuoka 418-0015 (JP); YASUNAGA Mitsuteru, Fujinomiya-shi, Shizuoka 418-0015 (JP); ASAWA Toshiki, Fujinomiya-shi, Shizuoka 418-0015 (JP)
(74) Representative: KIPA AB
(86) International application number: PCT/JP2024/025223
(87) International publication number: WO 2025/013930

(57) **Abstract**

Provided are: a penetration catheter that can effectively support a device to be inserted inside and can improve operability of the supported device while having high penetrability into a lesion of a vein; a treatment method; a guide wire; and a catheter system.

A penetration catheter 40 used for penetrating a lesion of a vein includes a second tube body 41 that has an outer diameter constant portion 45 having a constant outer diameter in a major axis direction, an outer tapered portion 46 having an outer diameter tapered from a distal end of the outer diameter constant portion 45 in a distal end direction, an inner diameter constant portion 47 having a constant inner diameter in the major axis direction, and an inner tapered portion 48 having an inner diameter tapered from a distal end of the inner diameter constant portion 47 in the distal end direction, in which an outer diameter of the outer diameter constant portion 45 is 2.7 ± 0.1 mm, an inner diameter of the inner diameter constant portion 47 is 1.6 ± 0.1 mm, an outer diameter of a most distal end of the tube body is 1.7 ± 0.1 mm, and an inner diameter of a most distal end of the second tube body 41 is 1.45 ± 0.1 mm.

## Description

### Technical Field

The present invention relates to a catheter, a treatment method, a guide wire, and a catheter system.

### Background Art

In the treatment of a blood vessel, when a guide wire is caused to pass through a lesion, in a state where the guide wire is inserted into and supported by a device (catheter) into which the guide wire is insertable, a procedure of causing the catheter to pass through the lesion together with the guide wire is performed (see, for example, Patent Literature 1).

Meanwhile, a support catheter, guiding catheter, or long sheath for an artery is diverted in treatment of deep vein thrombosis (DVT). The target blood vessel for deep vein thrombosis is a vein on the peripheral side of the inferior vena cava, and is, for example, an iliac vein, a femoral vein, a popliteal vein, or the like. The blood vessel diameter of a vein is generally greater than the blood vessel diameter of an artery. For example, the inner diameter of an artery accessed by the catheter is about 3 Fr to 8 Fr, whereas the inner diameter of a vein accessed by the catheter is about 6 Fr to 24 Fr.

### Citation List

### Patent Literature

Patent Literature 1: JP 4906347 B1

### Summary of Invention

### Technical Problem

Since a catheter for accessing a lesion of a vein, the catheter being adapted to the blood vessel diameter and the lesion of the vein is not known, when a thin catheter for an artery is used in a vein, the catheter is likely to bend in the thick vein, and penetrability into a lesion may be lowered.

The present invention has been made to solve the above-described problem, and an object thereof is to provide a penetration catheter that can effectively support a device to be inserted inside and can improve operability of the supported device while having high penetrability into a lesion of a vein; a treatment method; a guide wire that has high penetrability into a lesion of a vein; and a catheter system.

### Solution to Problem

The above object is achieved by the aspect described in (1) below.
(1) A penetration catheter according to the present invention is a penetration catheter used for penetrating a lesion of a vein, the penetration catheter including: a tube body that has an outer diameter constant portion having a constant outer diameter in a major axis direction, an outer tapered portion having an outer diameter tapered from a distal end of the outer diameter constant portion in a distal end direction, an inner diameter constant portion having a constant inner diameter in the major axis direction, and an inner tapered portion having an inner diameter tapered from a distal end of the inner diameter constant portion in the distal end direction, in which an outer diameter of the outer diameter constant portion is 2.7 mm, an inner diameter of the inner diameter constant portion is 1.6 mm, an outer diameter of a most distal end of the tube body is 1.6 mm, and an inner diameter of a most distal end of the tube body is 1.45 mm.

### Advantageous Effects of Invention

The penetration catheter described in (1) has a large wall thickness defined by the outer diameter constant portion and the inner diameter constant portion and high rigidity, and the most distal end of the outer tapered portion is thinned, so that penetrability into a lesion is high and a lesion that is difficult to penetrate can be penetrated by the penetration catheter itself. A device that is inserted into the penetration catheter can be effectively supported with high rigidity, and the operability of the supported device can be improved.

(2) In the penetration catheter described in (1), the tube body may have a curved portion that bends at an angle of 20 degrees to 30 degrees at a distal portion. Thus, since the penetration catheter can adjust the direction of the distal end by rotating the penetration catheter, operability can be improved.

(3) A treatment method for achieving the above object is a treatment method of penetrating a lesion of a vein, including: inserting an inner catheter into a penetration catheter, the penetration catheter having a tube body that has an outer diameter constant portion having a constant outer diameter in a major axis direction, an outer tapered portion having an outer diameter tapered from a distal end of the outer diameter constant portion in a distal end direction, an inner diameter constant portion having a constant inner diameter in the major axis direction, and an inner tapered portion having an inner diameter tapered from a distal end of the inner diameter constant portion in the distal end direction, in which an outer diameter of the outer diameter constant portion is 2.7 mm, an inner diameter of the inner diameter constant portion is 1.6 mm, an outer diameter of a most distal end of the tube body is 1.6 mm, and an inner diameter of a most distal end of the tube body is 1.45 mm; and penetrating the penetration catheter into a lesion of a vein together with a guide wire and the inner catheter in a state where the guide wire is inserted into the inner catheter. The penetration catheter used in this treatment method has a large wall thickness defined by the outer diameter constant portion and the inner diameter constant portion of the tube body and high rigidity, and the most distal end of the outer tapered portion is thinned, so that penetrability into a lesion is high. Therefore, according to this treatment method, a lesion that is difficult to penetrate can be penetrated by the penetration catheter itself. According to this treatment method, the inner catheter and the guide wire that are inserted into the penetration catheter can be effectively supported with high rigidity, and the operability of the supported devices can be improved.

(4) A guide wire for achieving the above object is a guide wire including: an elongated core wire that is formed of a metal; and a coating layer that covers at least a distal end of the core wire and is more flexible than the core wire, in which the core wire has a core wire outer diameter constant portion having a constant outer diameter, and a core wire tapered portion having an outer diameter tapered from a distal end of the core wire outer diameter constant portion to a most distal end of the core wire, a portion that has the core wire tapered portion in the guide wire is linearly formed, and in a load test for measuring a load when the guide wire is pushed in a distal end direction in a state where a distal end of the guide wire abuts against a receiving portion subjected to non-slip processing, a measured load exceeds 0.04 N when the guide wire is pushed in 0.05 mm, exceeds 0.05 N when the guide wire is pushed in 0.1 mm, and exceeds 0.05 N when the guide wire is pushed in 0.2 mm. Since the guide wire has a high load in the load test, penetrability into a lesion is high.

(5) A guide wire for achieving the above object is a guide wire including: an elongated core wire that is formed of a metal; and a coating layer that covers at least a distal end of the core wire and is more flexible than the core wire, in which the core wire has a core wire outer diameter constant portion having a constant outer diameter, and a core wire tapered portion having an outer diameter tapered from a distal end of the core wire outer diameter constant portion to a most distal end of the core wire, the guide wire has a guide wire distal end of a most distal end, a guide wire curved portion located on a proximal end side of the guide wire distal end and curved, and a linear guide wire linear portion located on a proximal end side of the guide wire curved portion, and has a distal end curve angle of 25 degrees to 40 degrees, the distal end curve angle being an angle at which a line segment connecting the guide wire distal end and the curved base portion is inclined with respect to an extension line of the guide wire linear portion, and in a load test for measuring a load when a distal end of the guide wire abuts against a receiving portion subjected to non-slip processing and the guide wire is pushed in a distal end direction in a state where the guide wire is inserted into the inner catheter and the inner catheter is inserted into a penetration catheter, a measured load exceeds 0.02 N when the guide wire is pushed in 0.2 mm, and exceeds 0.04 N when the guide wire is pushed in 2.0 mm. Since the guide wire has the guide wire curved portion, the direction of the distal end can be adjusted by rotating the guide wire, and operability can be improved. Since the guide wire has a high load in the load test, penetrability into a lesion is high.

(6) A catheter system for achieving the above object is a catheter system including: a penetration catheter used for penetrating a lesion of a vein; an inner catheter insertable into the penetration catheter; and the guide wire described in (4) insertable into the inner catheter, in which in a load test for measuring a load when the catheter system is pushed in a distal end direction in a state where the inner catheter is inserted into the penetration catheter, the guide wire is inserted into the inner catheter, and a distal end of the guide wire abuts against a receiving portion subjected to non-slip processing, a measured load exceeds 0.1 N when the catheter system is pushed in 0.2 mm. Since the catheter system has a high load in the load test, penetrability into a lesion is high.

(7) A catheter system for achieving the above object is a catheter system including: a penetration catheter used for penetrating a lesion of a vein; an inner catheter insertable into the penetration catheter; and the guide wire described in (5) insertable into the inner catheter, in which in a load test for measuring a load when the catheter system is pushed in a distal end direction in a state where the inner catheter is inserted into the penetration catheter, the guide wire is inserted into the inner catheter, and a distal end of the guide wire abuts against a receiving portion subjected to non-slip processing, a measured load exceeds 0.02 N when the catheter system is pushed in 0.2 mm, and exceeds 0.04 N when the catheter system is pushed in 2.0 mm. Since the guide wire in the catheter system has the guide wire curved portion, the direction of the distal end can be adjusted by rotating the guide wire, and operability can be improved. Since the catheter system has a high load in the load test, penetrability into a lesion is high.

### Brief Description of Drawings

Fig. 1 is a plan view illustrating a state where a catheter system is assembled.
Fig. 2 is a plan view illustrating a state where the catheter system is separated.
Fig. 3 is a cross-sectional view of a distal portion of a penetration catheter according to the present embodiment.
Fig. 4 is a cross-sectional view taken along line A-A in Fig. 1.
Fig. 5 is a cross-sectional view illustrating a guide wire, where Fig. 5(A) illustrates a first example and Fig. 5(B) illustrates a second example.
Fig. 6 is a schematic diagram illustrating a state where a loading test is performed, where Fig. 6(A) illustrates a state where a load test of the catheter system is performed and Fig. 6(B) illustrates a state where a load test of the guide wire is performed.
Fig. 7 is a graph showing a load with respect to a stroke in a loading test using the guide wire of the first example.
Fig. 8 is a graph showing a load with respect to a stroke in which a part of Fig. 7 is enlarged.
Fig. 9 is a graph showing a load with respect to a stroke in a loading test using the guide wire of the second example.
Fig. 10 is a graph showing a load with respect to a stroke in which a part of Fig. 9 is enlarged.
Fig. 11 is a graph showing a load with respect to a stroke in a loading test of a single guide wire.

### Description of Embodiments

Hereinafter, an embodiment of the present invention will be described with reference to the drawings. Note that dimensional ratios in the drawings may be exaggerated and different from actual ratios for convenience of description. In the following description, a side on which a catheter is operated is referred to as a "proximal end side", and a side to be inserted into a living body is referred to as a "distal end side".

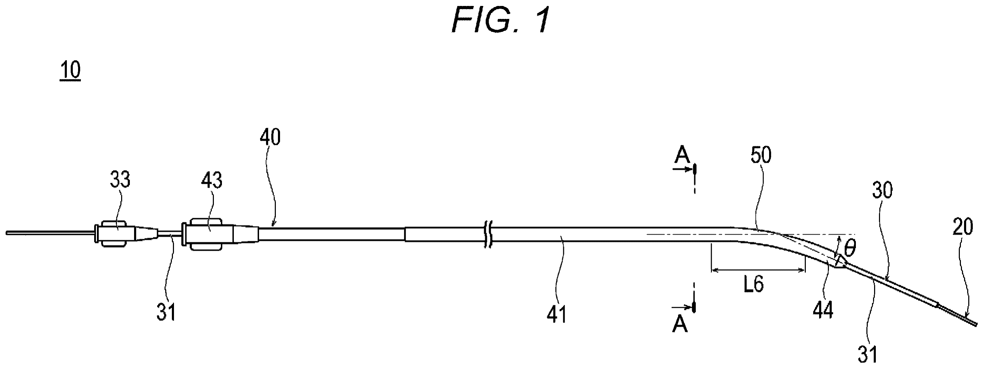

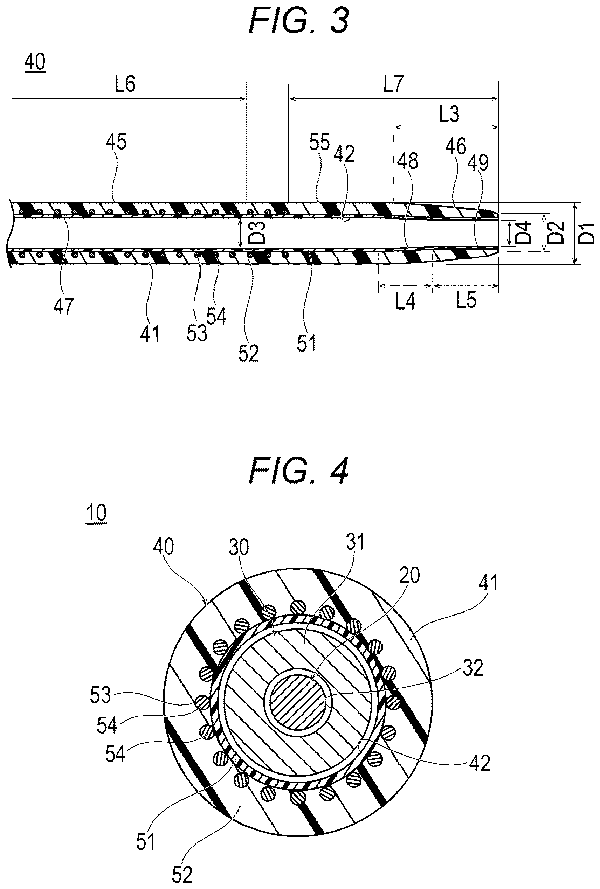

As shown in Figs. 1 to 4, a penetration catheter 40 according to the embodiment of the present invention constitutes a catheter system 10 together with other devices, and is used for penetrating a lesion in treatment of deep vein thrombosis (DVT). The catheter system 10 is inserted from a vein in a thigh or a knee, or a more peripheral vein and advanced in a blood vessel to be treated. The blood vessel to be treated is a vein (for example, an iliac vein, a femoral vein, a popliteal vein, or the like) on the peripheral side of the inferior vena cava.

The catheter system 10 includes the penetration catheter 40, an inner catheter 30 that is insertable into the penetration catheter 40, and a guide wire 20 that is insertable into the inner catheter 30.

The penetration catheter 40 is a catheter having high penetrability so that the penetration catheter 40 itself can be used for penetrating a lesion of a vein while supporting the inner catheter 30 and the guide wire 20 that are inserted therein. The penetration catheter 40 has a second tube body 41 (tube body) in which a second lumen 42 into which the inner catheter 30 is insertable is formed, and a second hub 43 that is fixed to a proximal portion of the second tube body 41. The penetration catheter 40 supports the inner catheter 30 inserted into the second lumen 42 and the guide wire 20 inserted into the inner catheter 30, and can penetrate a lesion together with the inner catheter 30 and the guide wire 20 while dilating the lesion. Note that when the catheter system 10 penetrates the lesion, the penetration catheter 40 may completely penetrate or partially penetrate the lesion. A hemostasis valve may be attached instead of the second hub 43.

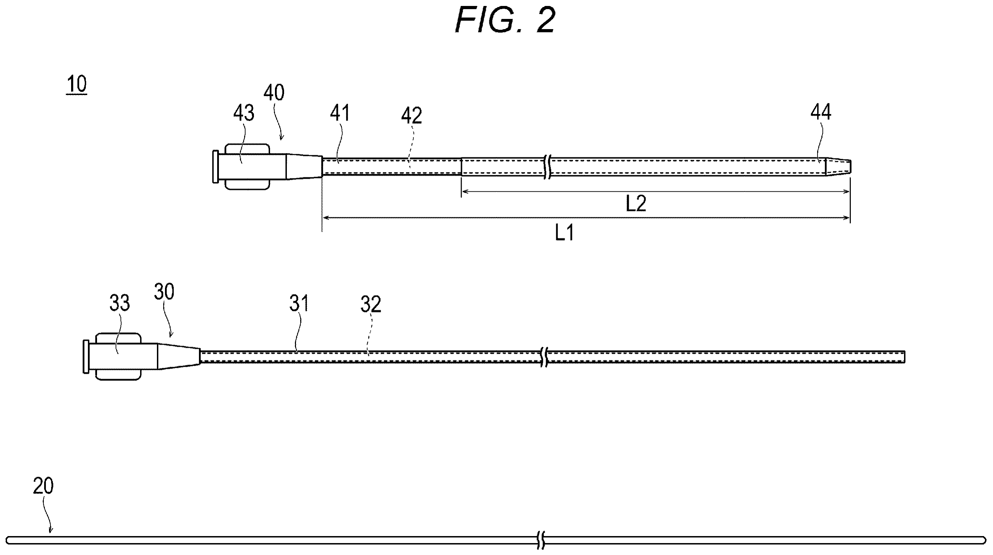

The outer peripheral surface of at least the distal portion of the second tube body 41 may be covered with a lubricious coat 44 (see Figs. 1 and 2). The lubricious coat 44 is covered in a range from the most distal end of the second tube body 41 to a predetermined distance L2 (for example, 400 mm) in a proximal end direction. The lubricious coat 44 is, for example, a hydrophilic polymer, and examples thereof include a cellulose-based polymer substance, a polyethylene oxide-based polymer substance, a maleic anhydride-based polymer substance (for example, a maleic anhydride copolymer such as a methyl vinyl ether-maleic anhydride copolymer), an acrylamide-based polymer substance (for example, a block copolymer of polyacrylamide and glycidyl methacrylate-dimethylacrylamide), watersoluble nylon, polyvinyl alcohol, polyvinyl pyrrolidone, and derivatives thereof. The hydrophilic polymer forms a strong water fixing layer on the surface thereof, exhibits high affinity to blood in a blood vessel and a wall surface of the blood vessel, and exhibits low friction. Alternatively, the lubricious coat 44 may be a fluorine-based resin such as polytetrafluoroethylene (PTFE), a low friction material such as high density polyethylene (HDPE), or the like. Meanwhile, a thrombus formed in a vein tends to be formed longer in a length direction of the blood vessel than a thrombus formed in an artery. Therefore, the fact that the outer peripheral surface of the second tube body 41 has low friction is very effective when the second tube body 41 penetrates a vein thrombus.

The second tube body 41 has an outer diameter constant portion 45 having a substantially constant outer diameter, an outer tapered portion 46 having an outer diameter tapered from a distal end of the outer diameter constant portion 45 toward a distal end direction, an inner diameter constant portion 47 having a substantially constant inner diameter, an inner tapered portion 48 having an inner diameter tapered from a distal end of the inner diameter constant portion 47 toward the distal end direction, and a distal end inner diameter constant portion 49 having a substantially constant inner diameter from a distal end of the inner tapered portion 48 toward the distal end direction. The second tube body 41 has a curved portion 50 in a range of a length L6 of a distal portion of the outer diameter constant portion 45. The length L6 is not particularly limited, but is, for example, 10 mm. A curve angle θ of the curved portion 50 is not particularly limited, but is preferably 20 degrees to 30 degrees, for example, 30 degrees. Note that the second tube body 41 may not have the curved portion 50.

The second tube body 41 is formed of an inner layer 51 forming an inner surface of the second tube body 41, an outer layer 52 forming an outer surface of the second tube body 41, and a reinforcing body 53 located between the inner layer 51 and the outer layer 52. The second tube body 41 has a flexible portion 55 on the distal end side of a region where the reinforcing body 53 is disposed, the reinforcing body 53 being not disposed in the flexible portion 55. A length L7 of the flexible portion 55 in the major axis direction is 20 mm to 30 mm.

The reinforcing body 53 is to reinforce the second tube body 41, and has a plurality of (for example, 16) reinforcing wires 54. The material of the outer layer 52 or the inner layer 51 is configured to enter a gap between the plurality of reinforcing wires 54 in the reinforcing body 53. Examples of the reinforcing body 53 include those in which the reinforcing wires 54 are meshed in a tubular shape. Note that the reinforcing body 53 may be formed by winding one or more reinforcing wires 54 in a spiral shape (coil shape). The reinforcing wire 54 is made of a metal such as stainless steel or NiTi. The cross-sectional shape of the reinforcing wire 54 is not particularly limited, and is, for example, a rectangular shape, a square shape, a circular shape, an oval shape, an elliptical shape, or the like. One reinforcing wire 54 may be formed by bundling two or more wire materials. The second tube body 41 having the reinforcing body 53 can ensure sufficient rigidity and strength without increasing the wall thickness, that is, while making the inner diameter of the second tube body 41 relatively large.

The inner layer 51 is preferably formed of a low friction material. Thus, when devices such as the inner catheter 30 and the guide wire 20 are inserted into and removed from the second lumen 42, the inner layer 51 enables these devices to move in the major axis direction with a small sliding resistance, so that operability is improved. Examples of the low friction material include fluorine-based resins such as polytetrafluoroethylene (PTFE) and a tetrafluoroethylene-perfluoroalkoxyethylene copolymer (PFA).

Examples of the constituent material of the outer layer 52 include polystyrene, polyolefin, polyurethane, polyester, polyamide, and various thermoplastic elastomers such as styrene, polyolefin, polyurethane, polyester, polyamide, polybutadiene, trans-polyisoprene, fluororubber, and chlorinated polyethylene-based thermoplastic elastomers, and one or a combination of two or more of these materials (polymer alloy, polymer blend, laminate, or the like) can be exemplified.

An effective length L1 of the penetration catheter 40 is not particularly limited, but is preferably 650 mm to 900 mm, for example, 650 mm. The effective length L1 of the penetration catheter 40 is a length of a portion insertable into a blood vessel or a tubular device. In the present embodiment, the effective length L1 of the penetration catheter 40 is a length from the most distal end of the second hub 43 or a kink protector at the proximal portion to the most distal end of the second tube body 41.

An outer diameter D1 of the outer diameter constant portion 45 is 2.7 ± 0.1 mm, an outer diameter D2 of the most distal end of the outer tapered portion 46 is 1.7±0.1 mm, an inner diameter D3 of the inner diameter constant portion 47 is 1.6 ± 0.1 mm, and an inner diameter D4 of the distal end inner diameter constant portion 49 is 1.45 ± 0.1 mm. A wall thickness T at a position where the outer diameter constant portion 45 and the inner diameter constant portion 47 overlap is 0.55 mm. When the inner diameters D3 and D4 are smaller than this wall thickness, the frictional resistance with the inner catheter 30 is large, and when the inner diameters D3 and D4 are larger than this wall thickness, the penetration catheter 40 is easily kinked. When the outer diameters D1 and D2 are smaller than this wall thickness, kinking is likely to occur, and when the outer diameters D1 and D2 are larger than this wall thickness, bending is difficult at the curved portion 50. A length L3 of the outer tapered portion 46 in the major axis direction is 12 mm, a length L4 of the inner tapered portion 48 in the major axis direction is 3 mm, and a length L5 of the distal end inner diameter constant portion 49 in the major axis direction is 7 mm.

The inner catheter 30 is a catheter that fills a space between the penetration catheter 40 and the guide wire 20 to increase the rigidity of the catheter system 10 and improve the penetrability, suppresses deflection of the guide wire 20 inside the penetration catheter 40 and the inner catheter 30, and dilates a gap of a lesion formed by the guide wire 20 that has entered the lesion. The inner catheter 30 has a first tube body 31 in which a first lumen 32 into which the guide wire 20 is insertable is formed, and a first hub 33 that is fixed to a proximal portion of the first tube body 31. The inner catheter 30 covers and supports the guide wire 20 inserted into the first lumen 32 and penetrates a lesion together with the guide wire 20 and the penetration catheter 40.

The outer diameter of the first tube body 31 is not particularly limited, but is preferably a size that allows smooth sliding while being in close contact with the second lumen 42 of the penetration catheter 40, and is, for example, 1.4 mm. The inner diameter of the first tube body 31 is preferably a size that allows the guide wire 20 inserted into the first lumen 32 to slide while being in close contact with the first lumen 32, and is, for example, 1.0 mm. As an example, the inner catheter 30 may be NaviCross (registered trademark) manufactured by Terumo Corporation into which the guide wire 20 having an outer diameter of 0.035 inches (0.9 mm) is insertable.

The guide wire 20 preferably has high penetrability so as to be able to penetrate a lesion of a vein. It is preferable that the guide wire 20 has higher rigidity of the distal portion than that of the general guide wire 20 so as to obtain high penetrability. For example, the guide wire 20 may be a wire obtained by removing a flexible distal portion (a range of 20 mm to 30 mm in the proximal end direction from the most distal end) of Glidewire Advantage (registered trademark) manufactured by Terumo Corporation having an outer diameter of 0.035 inches (0.9 mm).

As illustrated in Fig. 1, the catheter system 10 is used to cause the inner catheter 30 and the penetration catheter 40 as well as the guide wire 20 to penetrate a lesion of a vein (for example, an organized thrombus) in a state where the penetration catheter 40, the inner catheter 30, and the guide wire 20 are combined.

As described above, the penetration catheter 40 according to the present embodiment is the penetration catheter 40 used for penetrating a lesion of a vein including the second tube body 41 that has the outer diameter constant portion 45 having a constant outer diameter in a major axis direction, the outer tapered portion 46 having an outer diameter tapered from a distal end of the outer diameter constant portion 45 in a distal end direction, the inner diameter constant portion 47 having a constant inner diameter in the major axis direction, and the inner tapered portion 48 having an inner diameter tapered from a distal end of the inner diameter constant portion 47 in the distal end direction, in which an outer diameter of the outer diameter constant portion 45 is 2.7 ± 0.1 mm, an inner diameter of the inner diameter constant portion 47 is 1.6 ± 0.1 mm, an outer diameter of a most distal end of the tube body is 1.7 ± 0.1 mm, and an inner diameter of a most distal end of the second tube body 41 is 1.45 ± 0.1 mm. Thus, the penetration catheter 40 has a large wall thickness defined by the outer diameter constant portion 45 and the inner diameter constant portion 47 and high rigidity, and the most distal end of the outer tapered portion 46 is thinned, so that penetrability into a lesion is high and a lesion that is difficult to penetrate (for example, an organized thrombus) can be penetrated by the penetration catheter 40 itself. A device (the inner catheter 30 or the guide wire 20) that is inserted into the penetration catheter 40 can be effectively supported with high rigidity, and the operability of the supported device (inner catheter 30 or the guide wire 20) can be improved.

The second tube body 41 has the curved portion 50 that bends at an angle of 20 degrees to 30 degrees at the distal portion. Thus, since the penetration catheter 40 can adjust the direction of the distal end by rotating the penetration catheter, operability is improved.

A treatment method in the present embodiment is a treatment method of penetrating a lesion of a vein, including: inserting an inner catheter 30 into a penetration catheter 40, the penetration catheter 40 having a second tube body 41 that has an outer diameter constant portion 45 having a constant outer diameter in a major axis direction, an outer tapered portion 46 having an outer diameter tapered from a distal end of the outer diameter constant portion 45 in a distal end direction, an inner diameter constant portion 47 having a constant inner diameter in the major axis direction, and an inner tapered portion 48 having an inner diameter tapered from a distal end of the inner diameter constant portion 47 in the distal end direction, in which an outer diameter of the outer diameter constant portion 45 is 2.7 ± 0.1 mm, an inner diameter of the inner diameter constant portion 47 is 1.6 ± 0.1 mm, an outer diameter of a most distal end of the second tube body 41 is 1.7 ± 0.1 mm, and an inner diameter of a most distal end of the second tube body 41 is 1.45 ± 0.1 mm; and penetrating the penetration catheter 40 into a lesion of a vein together with a guide wire 20 and the inner catheter 30 in a state where the guide wire 20 is inserted into the inner catheter 30. The penetration catheter 40 used in this treatment method has a large wall thickness defined by the outer diameter constant portion 45 and the inner diameter constant portion 47 of the second tube body 41 and high rigidity, and the most distal end of the outer tapered portion 46 is thinned, so that penetrability into a lesion is high. Therefore, according to this treatment method, a lesion that is difficult to penetrate (for example, an organized thrombus) can be penetrated by the penetration catheter 40 itself. According to this treatment method, the inner catheter 30 and the guide wire 20 that are inserted into the penetration catheter 40 can be effectively supported with high rigidity, and the operability of the supported devices can be improved.

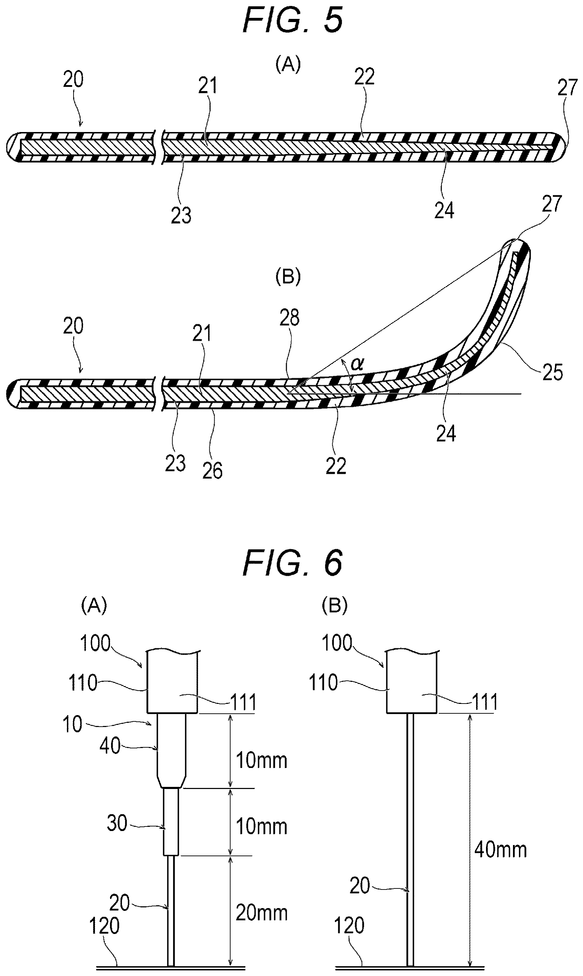

As illustrated in Fig. 5(A), a first example of the guide wire 20 includes an elongated core wire 21 and a coating layer 22 covering the core wire 21.

The core wire 21 extends over substantially the entire length of the guide wire 20. The core wire 21 is formed of one continuous wire material. The shape of the cross section perpendicular to the major axis of the core wire 21 is circular, but may not be circular.

The core wire 21 includes a core wire outer diameter constant portion 23 and a core wire tapered portion 24 disposed from a distal end of the core wire outer diameter constant portion 23 to a most distal end of the core wire 21. The core wire outer diameter constant portion 23 has a substantially constant outer diameter along the major axis. The core wire tapered portion 24 has an outer diameter that gradually decreases from the distal end of the core wire outer diameter constant portion 23 toward the distal end direction. The outer diameter of the core wire outer diameter constant portion 23 is, for example, 0.65 mm to 0.75 mm. The outer diameter of the most distal end of the core wire tapered portion 24 is, for example, 0.14 mm to 0.30 mm.

The core wire outer diameter constant portion 23 imparts desirable pushability to the guide wire 20 with a constant outer diameter. The core wire tapered portion 24 gradually decreases the rigidity of the guide wire 20 from the core wire outer diameter constant portion 23 toward the distal end direction. Thus, the guide wire 20 can suppress the occurrence of local bending such as a kink or damage.

As the material forming the core wire 21, a metal material can be used, and examples thereof include superelastic alloys such as a Ni-Ti-based alloy, a Ni-Al-based alloy, and a Cu-Zn-based alloy, and alloys containing these alloys.

The coating layer 22 covers at least the distal end of the core wire 21. Thus, the coating layer 22 protects the outer surface of the core wire 21 and improves safety when the guide wire 20 is inserted into a body lumen such as a blood vessel. Therefore, the material forming the coating layer 22 is preferably a material having high flexibility, and may be, for example, a polyolefin such as polyurethane, polyethylene, or polypropylene, a thermoplastic elastomer such as polyvinyl chloride, polyester (PET, PBT, or the like), polyamide, polyimide, polystyrene, a silicone resin, a polyurethane elastomer, a polyester elastomer, or a polyamide elastomer, various rubber materials such as latex rubber or silicone rubber, a fluororesin, and a composite material obtained by combining two or more thereof. The outer diameter of the coating layer 22 is, for example, 0.60 mm to 0.89 mm. Alternatively, the whole may be covered with a resin obtained by mixing polyurethane and tungsten, or the distal end may be covered with a resin obtained by mixing polyurethane and tungsten, and the proximal end side may be covered with a fluororesin such as PTFE.

The coating layer 22 may be covered with a lubricating layer (not illustrated). The lubricating layer is formed of a hydrophilic polymer that reduces friction. The hydrophilic polymer forms a strong water fixing layer on the surface thereof, exhibits high affinity to blood in a blood vessel and a wall surface of the blood vessel, and exhibits low friction (low friction coefficient). The lubricating layer may cover the entire length as long as it covers at least the distal end of the coating layer 22.

As in a second example illustrated in Fig. 5(B), the guide wire 20 may have a guide wire curved portion 25 at the distal portion where the core wire tapered portion 24 is located. The guide wire 20 has a guide wire linear portion 26 on the proximal end side of the guide wire curved portion 25. The guide wire 20 has a guide wire distal end 27 at the most distal end, and has a curved base portion 28 between the guide wire linear portion 26 and the guide wire curved portion 25. A distal end curve angle α, which is an angle at which a line segment connecting the curved base portion 28 and the guide wire distal end 27 is inclined with respect to an extension line of the guide wire linear portion 26, is not particularly limited, but is preferably 25 degrees to 40 degrees. The length from the curved base portion 28 to the guide wire distal end 27 along the major axis of the guide wire 20 is not particularly limited, but is preferably 3 mm to 5 mm.

The end surface of the guide wire distal end 27 has a curvature and is formed in a substantially hemispherical shape. Therefore, as compared with a case where the end surface is a horizontal surface, a contact object can be brought into contact at a point, so that the contact object is less likely to slip and easily enter the gap. The end surface of the guide wire distal end 27 may be covered with a lubricating layer or may not be covered. When the end surface of the guide wire distal end 27 does not have the lubricating layer while the guide wire 20 has the lubricating layer on the surface, the guide wire distal end 27 becomes less slippery and easily enters the gap.

As an example of a combination of the penetration catheter 40, the inner catheter 30, and the guide wire 20 of the catheter system 10, the penetration catheter 40 may have the curved portion 50, the inner catheter 30 may not have a curved portion, and the guide wire 20 may have the guide wire curved portion 25. As another example of the combination, the penetration catheter 40 may have the curved portion 50, the inner catheter 30 may not have a curved portion, and the guide wire 20 may not have the guide wire curved portion 25. As still another example of the combination, the penetration catheter 40 may not have the curved portion 50, the inner catheter 30 may not have a curved portion, and the guide wire 20 may not have the guide wire curved portion 25. When the penetration catheter 40 has the curved portion 50, the direction of the distal end of the penetration catheter 40 can be changed by rotating the penetration catheter, which is effective, for example, at the time of puncture. When the guide wire 20 has the guide wire curved portion 25, the direction of the distal end of the guide wire 20 can be changed by rotating the guide wire, and thus, for example, the guide wire 20 can be suppressed from erroneously entering into a side branch in a blood vessel. When all of the penetration catheter 40, the inner catheter 30, and the guide wire 20 do not have a curved portion, the catheter system 10 can obtain a high penetration force.

### <Loading test>

A load test of the catheter system 10 and the guide wire 20 was performed using a load testing device 100 illustrated in Fig. 6. As illustrated in Fig. 6(A), the load testing device 100 has a pressing portion 110 that is movable so as to apply a load downward and has a chuck mechanism 111 capable of fixing an elongated member, and a receiving portion 120 that is located below the pressing portion 110 and is capable of coming into contact with the distal end of the guide wire 20 so as not to slip. The receiving portion 120 was obtained by attaching a #600 (abrasive grain diameter: 28 µm) sandpaper in accordance with JIS standards to an upper surface of a stainless steel flat plate with a double-sided tape. As illustrated in Fig. 6(A), when the loading test was performed in a state where the guide wire 20 is combined with the penetration catheter 40 and the inner catheter 30, the chuck mechanism 111 fixed a position of 10 mm from the distal end to the proximal end side of the penetration catheter 40 not having the curved portion 50. Note that the chuck mechanism 111 could deform the penetration catheter 40 and the inner catheter 30 when the penetration catheter 40 was sandwiched, and could fix the penetration catheter 40, the inner catheter 30, and the guide wire 20. The distal end of the inner catheter 30 was at a position of 10 mm downward (distal end direction) from the distal end of the penetration catheter 40. The distal end of the guide wire 20 was at a position of 20 mm downward (distal end direction) from the distal end of the inner catheter 30. The loading test device 100 was capable of vertically moving the pressing portion 110 having the chuck mechanism 111 with respect to the receiving portion 120 that does not move. In the loading test, the pressing portion 110 was moved downward by 2 mm at 5 mm/min from a state where the distal end of the guide wire 20 was in contact with the receiving portion 120, and downward stroke and a load acting on the pressing portion 110 were measured.

As illustrated in Fig. 6(B), when a loading test is performed with the single guide wire 20, the chuck mechanism 111 fixes a position of 40 mm from the distal end of the guide wire 20 to the proximal end side.

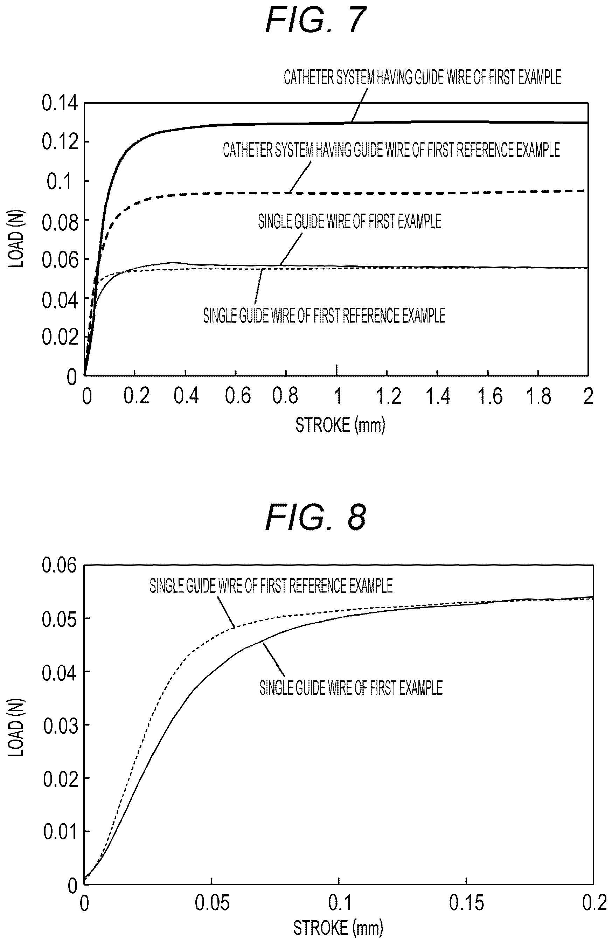

Figs. 7 and 8 show results of a load test of the guide wire 20 of the first example without the guide wire curved portion 25. A thick line in Fig. 7 is a test result of the guide wire 20 combined with the penetration catheter 40 and the inner catheter 30. A thick solid line is a test result of the guide wire 20 of the first example (the linear guide wire 20 without the guide wire curved portion 25) illustrated in Fig. 5(A), and a thick dotted line is a test result of the guide wire 20 as a first reference example. Unlike the first example, the guide wire 20 of the first reference example has the core wire 21 including a distal end outer diameter constant portion (not illustrated) having a constant outer diameter on the distal end side of the core wire tapered portion 24. The length of the distal end outer diameter constant portion in the major axis direction was about 20 mm. That is, the guide wire 20 of the first example had a shape in which the distal end outer diameter constant portion is removed and was formed to be short in the major axis direction by that removal amount, as compared with the guide wire 20 of the first reference example.

Thin lines in Figs. 7 and 8 are test results of the single guide wire 20. A thin solid line is a test result of the single guide wire 20 of the first example, and a thin dotted line is a test result of the single guide wire 20 of the first reference example. Fig. 8 shows the test results of the single guide wire 20 of Fig. 7 in an enlarged manner.

As indicated by the thick line in Fig. 7, in a state of being combined with the penetration catheter 40 and the inner catheter 30, the guide wire 20 of the first example obtained a higher load than the guide wire 20 of the first reference example. On the other hand, as indicated by the thin lines in Figs. 7 and 8, in the test of the single guide wire 20, the guide wire 20 of the first example had no large difference in the load as compared with the guide wire 20 of the first reference example. That is, it was confirmed that when being combined with the penetration catheter 40 and the inner catheter 30, the guide wire 20 of the first example can obtain higher penetrability than the guide wire 20 of the first reference example.

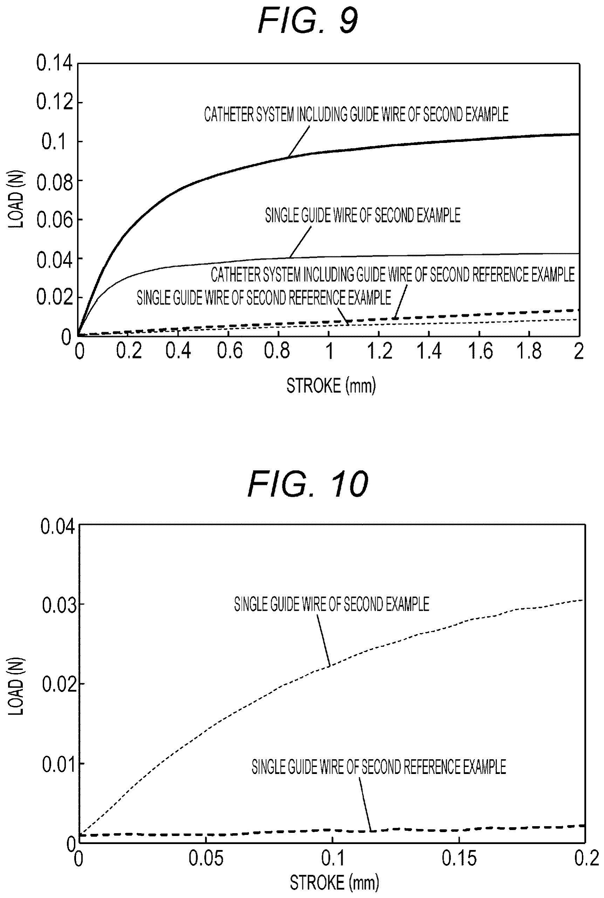

Figs. 9 and 10 show results of a load test of the guide wire 20 of the second example with the guide wire curved portion 25. A thick line in Fig. 9 is a test result of the guide wire 20 combined with the penetration catheter 40 and the inner catheter 30. A thick solid line is a test result of the guide wire 20 of the second example illustrated in Fig. 5(B), and a thick dotted line is a test result of the guide wire 20 as a second reference example. The distal end curve angle α of the guide wire 20 of the second example was 40 degrees. The length from the curved base portion 28 to the guide wire distal end 27 along the major axis of the guide wire 20 was 3 mm. The guide wire 20 of the second reference example had the guide wire curved portion 25 as in the second example, and had the core wire 21 including a distal end outer diameter constant portion having a constant outer diameter on the distal end side of the core wire tapered portion 24. The length of the distal end outer diameter constant portion in the major axis direction was about 20 mm. That is, the guide wire 20 of the second reference example was formed to be long in the major axis direction because the distal end outer diameter constant portion was present on the distal end side of the core wire tapered portion 24, as compared with the guide wire 20 of the second example. The guide wire curved portion 25 of the guide wire 20 of the second reference example had a J-shaped curved shape different from that of the second example, and had a radius of curvature larger than that of the guide wire curved portion 25 of the guide wire 20 of the second example. The distal end curve angle α of the guide wire 20 of the second reference example was 40 degrees. The length from the curved base portion 28 to the guide wire distal end 27 along the major axis of the guide wire 20 of the second reference example was 15 mm.

Thin lines in Figs. 9 and 10 are test results of the single guide wire 20. A thin solid line is a test result of the single guide wire 20 of the second example, and a thin dotted line is a test result of the single guide wire 20 of the second reference example. Fig. 10 shows the test result of the single guide wire 20 of Fig. 9 in an enlarged manner.

As indicated by the thick line in Fig. 9, in a state of being combined with the penetration catheter 40 and the inner catheter 30, the guide wire 20 of the second example obtained a higher load than the guide wire 20 of the second reference example. As indicated by the thin lines in Figs. 9 and 10, also in the test of the single guide wire 20, the guide wire 20 of the second example obtained a higher load than the guide wire 20 of the second reference example. That is, it was confirmed that not only when being combined with the penetration catheter 40 and the inner catheter 30 but also in the case of the single guide wire, the guide wire 20 of the second example can obtain higher penetrability than the guide wire 20 of the second reference example. Note that it was confirmed that the guide wire 20 of the second example has a larger increase amount of the load in a state of being combined with the penetration catheter 40 and the inner catheter 30 than in a state of the single guide wire as compared with the second reference example, and can obtain a higher penetration force than the second reference example.

The load of the guide wire 20 of the second example in Figs. 9 and 10 was smaller than the load of the guide wire 20 of the first example in Figs. 7 and 8. This is considered to be because the guide wire 20 of the second example has the guide wire curved portion 25 which is not included in the guide wire 20 of the first example, and thus the position in contact with the receiving portion 120 is shifted in the horizontal direction from immediately below the pressing portion 110.

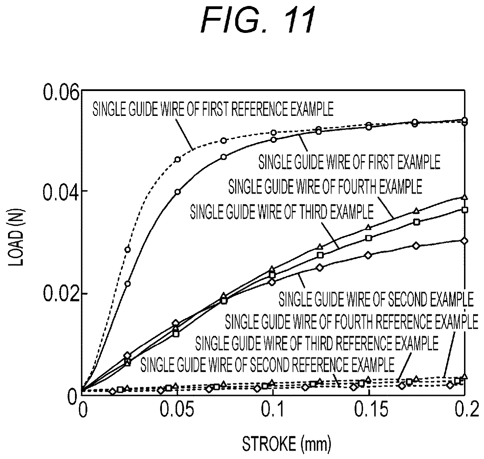

Fig. 11 shows results of a load test of the single guide wire 20. Solid lines in Fig. 11 are test results of the first to fourth examples of the guide wire 20 having the core wire 21 not including the distal end outer diameter constant portion having a constant outer diameter on the distal end side of the core wire tapered portion 24. Dotted lines in Fig. 11 are test results of the first to fourth reference examples of the guide wire 20 having the core wire 21 including the distal end outer diameter constant portion having a constant outer diameter on the distal end side of the core wire tapered portion 24.

The solid lines in Fig. 11 indicate test results of the third example in which the distal end curve angle α is 30 degrees and the fourth example in which the distal end curve angle α is 25 degrees in addition to the first example in which the distal end curve angle α is 0 degrees and the second example in which the distal end curve angle α is 40 degrees described above. The dotted lines in Fig. 11 indicate test results of the third reference example in which the distal end curve angle α is 30 degrees and the fourth reference example in which the distal end curve angle α is 25 degrees in addition to the first reference example in which the distal end curve angle α is 0 degrees and the second reference example in which the distal end curve angle α is 40 degrees described above. From the results of Fig. 11, it was confirmed that the load of the guide wire 20 of the first to fourth examples in which the distal end curve angle α is 25 degrees to 40 degrees is increased more than that of the guide wire 20 of the first to fourth reference examples, and high penetrability can be obtained.

As described above, the guide wire 20 of the first example is the guide wire 20 including: an elongated core wire 21 that is formed of a metal; and a coating layer 22 that covers the core wire 21 and is more flexible than the core wire 21, in which the core wire 21 has a core wire outer diameter constant portion 23 having a constant outer diameter, and a core wire tapered portion 24 having an outer diameter tapered from a distal end of the core wire outer diameter constant portion 23 to a most distal end of the core wire 21, a portion that has the core wire tapered portion 24 in the guide wire 20 is linearly formed, and in a load test for measuring a load when the guide wire 20 is pushed in a distal end direction in a state where a distal end of the guide wire 20 abuts against a receiving portion 120 subjected to non-slip processing, a measured load exceeds 0.04 N when the guide wire 20 is pushed in 0.05 mm, exceeds 0.05 N when the guide wire 20 is pushed in 0.1 mm, and exceeds 0.05 N when the guide wire 20 is pushed in 0.2 mm. Since the guide wire 20 of the first example has a high load in the load test, penetrability into a lesion is high.

The guide wire 20 of the second example is the guide wire 20 including: an elongated core wire 21 that is formed of a metal; and a coating layer 22 that covers the core wire 21 and is more flexible than the core wire 21, in which the core wire 21 has a core wire outer diameter constant portion 23 having a constant outer diameter, and a core wire tapered portion 24 having an outer diameter tapered from a distal end of the core wire outer diameter constant portion 23 to a most distal end of the core wire 21, the guide wire 20 has a guide wire distal end 27 of a most distal end, a guide wire curved portion 25 located on a proximal end side of the guide wire distal end 27 and curved, and a linear guide wire linear portion 26 located on a proximal end side of the guide wire curved portion 25, and has a distal end curve angle α of 25 degrees to 40 degrees, the distal end curve angle α being an angle at which a line segment connecting the guide wire distal end 27 and the curved base portion 28 is inclined with respect to an extension line of the guide wire linear portion 26, and in a load test for measuring a load when a distal end of the guide wire 20 abuts against a receiving portion 120 subjected to non-slip processing and the guide wire 20 pushed in a distal end direction in a state where the guide wire 20 is inserted into the inner catheter 30 and the inner catheter 30 is inserted into a penetration catheter 40, a measured load exceeds 0.02 N when the guide wire 20 is pushed in 0.2 mm, and exceeds 0.04 N when the guide wire 20 is pushed in 2.0 mm. Since the guide wire 20 of the second example has the guide wire curved portion 25, the direction of the distal end can be adjusted by rotating the guide wire 20, and operability can be improved. Since the guide wire 20 of the second example has a high load in the load test, penetrability into a lesion is high.

The catheter system 10 is the catheter system 10 including: a penetration catheter 40 used for penetrating a lesion of a vein; an inner catheter 30 insertable into the penetration catheter 40; and the guide wire 20 of the first example insertable into the inner catheter 30, in which in a load test for measuring a load when the catheter system 10 is pushed in a distal end direction in a state where the inner catheter 30 is inserted into the penetration catheter 40, the guide wire 20 is inserted into the inner catheter 30, and a distal end of the guide wire 20 abuts against a receiving portion 120 subjected to non-slip processing, a measured load exceeds 0.1 N when the catheter system 10 is pushed in 0.2 mm. Since the catheter system 10 has a high load in the load test, penetrability into a lesion is high.

The catheter system 10 is the catheter system 10 including: a penetration catheter 40 used for penetrating a lesion of a vein; an inner catheter 30 insertable into the penetration catheter 40; and the guide wire 20 of the second example insertable into the inner catheter 30, in which in a load test for measuring a load when the catheter system 10 is pushed in a distal end direction in a state where the inner catheter 30 is inserted into the penetration catheter 40, the guide wire 20 is inserted into the inner catheter 30, and a distal end of the guide wire 20 abuts against a receiving portion 120 subjected to non-slip processing, a measured load exceeds 0.02 N when the catheter system 10 is pushed in 0.2 mm, and exceeds 0.04 N when the catheter system 10 is pushed in 2.0 mm Since the guide wire 20 in the catheter system 10 has the guide wire curved portion 25, the direction of the distal end can be adjusted by rotating the guide wire 20, and operability can be improved. Since the catheter system 10 has a high load in the load test, penetrability into a lesion is high.

Note that the present invention is not limited to the embodiment described above, and those skilled in the art can make various modifications within the technical idea of the present invention. For example, a hub connected to the proximal portion of the tube body of each catheter may or may not be provided with a valve body or a port. In addition, a kink protector may be disposed so as to cover a space between the tube body and the hub of each catheter.

Note that the present application is based on Japanese Patent Application No. 2023-114344 filed on July 12, 2023, the entire disclosed content of which is incorporated herein by reference.

### Reference Signs List

- 10: Catheter system
- 20: Guide wire
- 21: Core wire
- 22: Coating layer
- 23: Core wire outer diameter constant portion
- 24: Core wire tapered portion
- 25: Guide wire curved portion
- 26: Guide wire linear portion
- 27: Guide wire distal end
- 28: Curved base portion
- 30: Inner catheter
- 40: Penetration catheter
- 41: Second tube body (tube body)
- 45: Outer diameter constant portion
- 46: Outer tapered portion
- 47: Inner diameter constant portion
- 48: Inner tapered portion
- 49: Distal end inner diameter constant portion
- 50: Curved portion
- 100: Load testing device
- 110: Pressing portion
- 120: Receiving portion
- α: Distal end curve angle

## Claims

1. A penetration catheter used for penetrating a lesion of a vein, the penetration catheter comprising:
a tube body that has an outer diameter constant portion having a constant outer diameter in a major axis direction, an outer tapered portion having an outer diameter tapered from a distal end of the outer diameter constant portion in a distal end direction, an inner diameter constant portion having a constant inner diameter in the major axis direction, and an inner tapered portion having an inner diameter tapered from a distal end of the inner diameter constant portion in the distal end direction, wherein
an outer diameter of the outer diameter constant portion is 2.7 ± 0.1 mm, an inner diameter of the inner diameter constant portion is 1.6 ± 0.1 mm, an outer diameter of a most distal end of the tube body is 1.7 ± 0.1 mm, and an inner diameter of a most distal end of the tube body is 1.45 ± 0.1 mm.

2. The penetration catheter according to claim 1, wherein the tube body has a curved portion that bends at an angle of 20 degrees to 30 degrees at a distal portion.

3. A treatment method of penetrating a lesion of a vein, comprising:
inserting an inner catheter into a penetration catheter, the penetration catheter having a tube body that has an outer diameter constant portion having a constant outer diameter in a major axis direction, an outer tapered portion having an outer diameter tapered from a distal end of the outer diameter constant portion in a distal end direction, an inner diameter constant portion having a constant inner diameter in the major axis direction, and an inner tapered portion having an inner diameter tapered from a distal end of the inner diameter constant portion in the distal end direction, in which an outer diameter of the outer diameter constant portion is 2.7 ± 0.1 mm, an inner diameter of the inner diameter constant portion is 1.6 ± 0.1 mm, an outer diameter of a most distal end of the tube body is 1.7 ± 0.1 mm, and an inner diameter of a most distal end of the tube body is 1.45 ± 0.1 mm; and penetrating the penetration catheter into a lesion of a vein together with a guide wire and the inner catheter in a state where the guide wire is inserted into the inner catheter.

4. A guide wire comprising: an elongated core wire that is formed of a metal; and a coating layer that covers at least a distal end of the core wire and is more flexible than the core wire, wherein
the core wire has a core wire outer diameter constant portion having a constant outer diameter, and a core wire tapered portion having an outer diameter tapered from a distal end of the core wire outer diameter constant portion to a most distal end of the core wire,
a portion that has the core wire tapered portion in the guide wire is linearly formed, and
in a load test for measuring a load when the guide wire is pushed in a distal end direction in a state where a distal end of the guide wire abuts against a receiving portion subjected to non-slip processing, a measured load exceeds 0.04 N when the guide wire is pushed in 0.05 mm, exceeds 0.05 N when the guide wire is pushed in 0.1 mm, and exceeds 0.05 N when the guide wire is pushed in 0.2 mm.

5. A guide wire comprising: an elongated core wire that is formed of a metal; and a coating layer that covers at least a distal end of the core wire and is more flexible than the core wire, wherein
the core wire has a core wire outer diameter constant portion having a constant outer diameter, and a core wire tapered portion having an outer diameter tapered from a distal end of the core wire outer diameter constant portion to a most distal end of the core wire,
the guide wire has a guide wire distal end of a most distal end, a guide wire curved portion located on a proximal end side of the guide wire distal end and curved, and a linear guide wire linear portion located on a proximal end side of the guide wire curved portion, and has a distal end curve angle α of 25 degrees to 40 degrees, the distal end curve angle α being an angle at which a line segment connecting the guide wire distal end and the curved base portion is inclined with respect to an extension line of the guide wire linear portion, and
in a load test for measuring a load when a distal end of the guide wire abuts against a receiving portion subjected to non-slip processing and the guide wire is pushed in a distal end direction in a state where the guide wire is inserted into the inner catheter and the inner catheter is inserted into a penetration catheter, a measured load exceeds 0.02 N when the guide wire is pushed in 0.2 mm, and exceeds 0.04 N when the guide wire is pushed in 2.0 mm.

6. A catheter system comprising: a penetration catheter used for penetrating a lesion of a vein; an inner catheter insertable into the penetration catheter; and the guide wire according to claim 4 insertable into the inner catheter, wherein
in a load test for measuring a load when the catheter system is pushed in a distal end direction in a state where the inner catheter is inserted into the penetration catheter, the guide wire is inserted into the inner catheter, and a distal end of the guide wire abuts against a receiving portion subjected to non-slip processing, a measured load exceeds 0.1 N when the catheter system is pushed in 0.2 mm.

7. A catheter system comprising: a penetration catheter used for penetrating a lesion of a vein; an inner catheter insertable into the penetration catheter; and the guide wire according to claim 5 insertable into the inner catheter, wherein
in a load test for measuring a load when the catheter system is pushed in a distal end direction in a state where the inner catheter is inserted into the penetration catheter, the guide wire is inserted into the inner catheter, and a distal end of the guide wire abuts against a receiving portion subjected to non-slip processing, a measured load exceeds 0.02 N when the catheter system is pushed in 0.2 mm, and exceeds 0.04 N when the catheter system is pushed in 2.0 mm.
